# EUROPÄISCHE PATENTSCHRIFT

(11) **EP 0 917 449 B1**
(45) Veröffentlichungstag und Bekanntmachung des Hinweises auf die Patenterteilung: **05.02.2003**
(21) Anmeldenummer: 96923805.4
(22) Anmeldetag: 31.07.1996
(51) Int. Cl.: A61B 17/74, A61B 17/86

(54) **VORRICHTUNG ZUM FIXIEREN ABGEBROCHENER HÜFTGELENKKÖPFE**
DEVICE FOR ATTACHING FRACTURED HIP-JOINT HEADS
DISPOSITIF DE FIXATION DE TETES D'ARTICULATION DE HANCHE FRACTUREES

(43) Veröffentlichungstag der Anmeldung: 26.05.1999
(73) Patentinhaber: SYNTHES AG CHUR, 7002 Chur (CH)
(72) Erfinder: FRIGG, Robert, CH-7270 Davos-Platz (CH); SCHWYN, Ronald, CH-7277 Davos-Glaris (CH)
(74) Vertreter: Lusuardi, Werther Giovanni, Dr.
(86) Internationale Anmeldenummer: CH9600270
(87) Internationale Veröffentlichungsnummer: WO98005263

(56) Entgegenhaltungen:
- EP-A- 0 257 118
- EP-A- 0 411 273
- EP-A- 0 491 138
- WO-A-91/09572
- DE-A- 4 106 876
- DE-C- 587 317
- DE-C- 757 951
- US-A- 2 121 193
- US-A- 3 996 931
- US-A- 4 103 683
- US-A- 4 441 492
- US-A- 5 269 686
- US-A- 5 324 292

## Beschreibung

Die Erfindung bezieht sich auf eine Vorrichtung zum Fixieren abgebrochener Hüftgelenkköpfe gemäss dem Oberbegriff des Patentanspruchs 1. Eine solche Vorrichtung ist aus der DE-A 41 06 876 bekannt; ihre Ankerschraube weist lediglich ein eingängiges Gewinde auf. Solche Vorrichtungen sind im allgemeinen als sogenannte "Hüftschrauben" bekannt. Allen bekannten Ausführungen ist der Nachteil gemeinsam, dass bei der Implantation ein unverhältnismässig grosser Knochenverlust entsteht, der einen späteren ev. notwenig werdenden zweiten Eingriff verunmöglicht oder stark erschwert.

Aus der US-A-5 269 686 ist ein Dentalimplantat mit einem viergängigen Gewinde bekannt. Schliesslich ist aus der EP-A 0 257 118 ein Marknagel mit rotationsstabil einführbarer Schenkelhalsschraube bekannt, welche aber lediglich ein eingängiges Gewinde aufweist.

Hier will die Erfindung Abhilfe schaffen. Der Erfindung liegt die Aufgabe zugrunde, eine Vorrichtung zum Fixieren abgebrochener Hüftgelenkköpfe zu schaffen, deren Implantation nur einen geringfügigen Knochenverlust verursacht und damit auch die Möglichkeit eines zweiten Eingriffs wahrt.
Die Erfindung löst die gestellte Aufgabe mit einer Vorrichtung, welche die Merkmale des Anspruchs 1 aufweist. Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung der Femurkopf - trotz Beibehaltung seiner axialen Verschiebbarkeit in Richtung des Schenkelhalses - rotationsstabil fixierbar ist. Sollte die Gleiteigenschaft der Ankerschraube in der Hülse erschwert werden, verhindert die mehrgängige Gewindepartie der Ankerschraube dank ihrer projizierten Kreisfläche ein Penetrieren des Femurkopfes. Die gleiche Projektionsfläche könnte zwar auch bei einer kleinen Gewindesteigung erreicht werden, jedoch ist in diesem Falle die Rotationsstabilität des Femurkopfes nicht gewährleistet. Die Ankerschraube wird durch axiale Kraft in den Knochen eingebracht. Je nach Knochenqualität erfolgt dies bei Hand oder mit einem Einschlaginstrument. Der weitere Vorteil gegenüber einer üblichen Hüftschraube ist, dass sich dank des steilen Spiralwinkels der Flügel, beim Einbringen kein Drehmoment auf den Femurkopf überträgt, was die Dislokation des Femurkopfes verhindert.

Die Erfindung und Weiterbildungen der Erfindung werden im folgenden anhand der teilweise schematischen Darstellung eines Ausführungsbeispiels noch näher erläutert.
Es zeigen:
Fig. 1 eine perspektivische Darstellung der demontierten erfindungsgemässen Vorrichtung;
Fig. 2 einen teilweisen Schnitt durch die im Femur implantierte erfindungsgemässen Vorrichtung; und
Fig. 3 einen Schnitt längs der Linie III-III in Fig. 2.

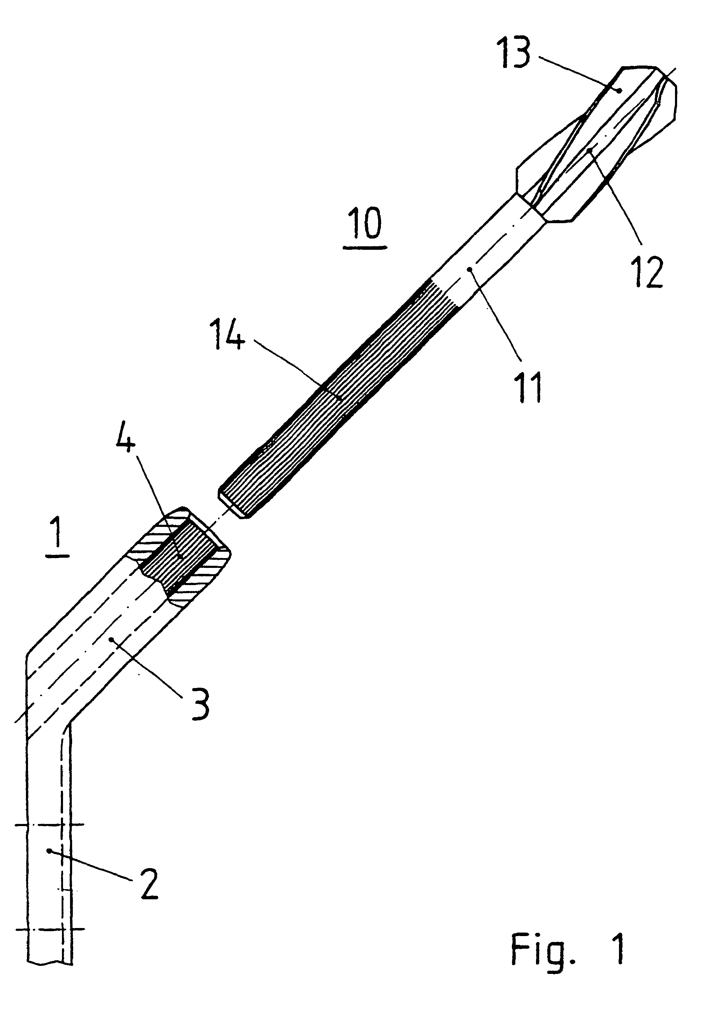

Die in Fig. 1 dargestellte erfindungsgemässen Vorrichtung umfasst im wesentlichen eine Winkelplatte 1, welche eine am Femur befestigbare Knochenplatte 2 und eine an der Knochenplatte winkelig anschliessende Hülse 3 umfasst. Die Vorrichtung umfasst ferner eine Ankerschraube 10, welche einen in die Hülse 3 einführbaren Schaft 11 und eine koaxial daran anschliessende Gewindepartie 12 umfasst.

Das Innere der Hülse 3 und das Äussere des Schaftes 11 sind mit Mitteln 14,4 - in der bevorzugten zeichnerisch dargestellten Ausführungsform in Form mehrerer axial auf dem Schaft 11 verlaufenden Längsrillen 14 und damit korrespondierenden, axial in der Hülse 3 verlaufenden Nuten 4 - versehen, welche zur gegenseitigen Rotationssicherung unter Beibehaltung der axialen Verschiebbarkeit dienen.

Die Gewindepartie 12 weist ein mehrgängiges - vorzugsweise viergängiges - Gewinde 13 auf. Die Steigung des Gewindes 13 beträgt mindestens 50 mm, vorzugsweise mindestens 80 mm.
Der Aussendurchmesser des Gewindes 13 beträgt 10 - 14 mm, vorzugsweise 11 - 13 mm.

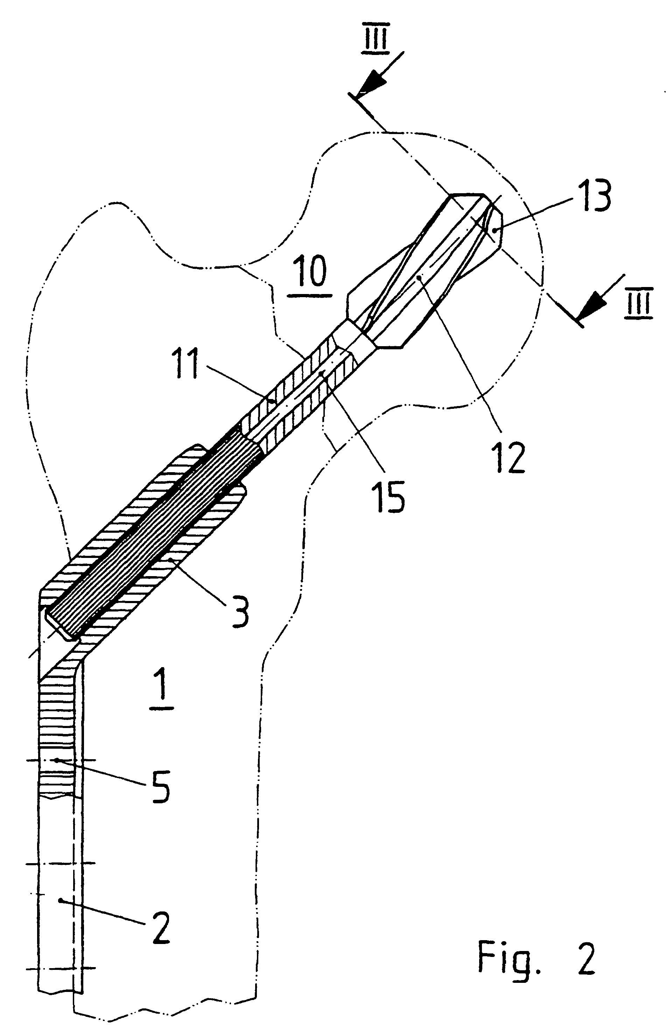

Wie in Fig. 2 dargestellt kann die Knochenplatte 2 mittels in die Bohrungen 5 einzuführender - zeichnerisch nicht dargestellter - unikortikaler, selbstbohrender Schrauben lateral am Femur befestigt werden, währenddem die Hülse 3 lateral der Schenkelhalsfraktur zu liegen kommt. Somit kann der Femurkopf mit Hilfe der Ankerschraube 10 mit dem Rest des Schenkelhalses rotationsstabil fixiert werden. Zentral in der Ankerschraube 10 ist ein Längskanal 15 angeordnet, der einen Führungsdraht aufnehmen kann.

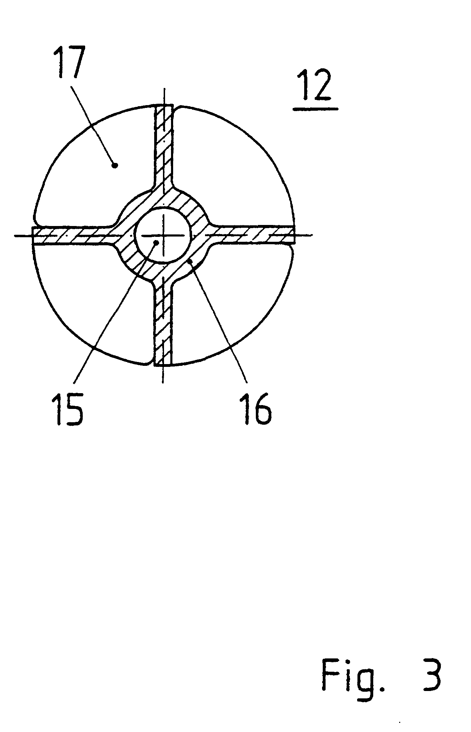

Wie in Fig. 3 dargestellt besteht die Gewindepartie 12 aus einem Kern 16 mit dem Längskanal 15 und spiralförmig um den Kern 16 laufenden Flügeln 17. Die Spiralwinkel der einzelnen Flügel sind so gross bemessen, dass sie in der axialen Projektion annähernd eine Kreisfläche ergeben. Die Querschnittsfläche der Gewindepartie 12 beträgt höchstens 55 mm², vorzugsweise höchstens 35 mm² und mindestens 10 mm², vorzugsweise mindestens 20 mm².
Die Flügel 17 weisen eine Dicke von maximal 2,0 mm, vorzugsweise von maximal 1,2 mm und von mindestens 0,5 mm, vorzugsweise von mindestens 0,8 mm auf.

## Patentansprüche

1. Vorrichtung zum Fixieren abgebrochener Hüftgelenkköpfe mit
A) einer Winkelplatte (1), welche eine am Femur befestigbare Knochenplatte (2) und eine an der Knochenplatte winkelig anschliessende Hülse- (3) umfasst;
B) einer Ankerschraube (10), welche einen in die Hülse (3) einführbaren Schaft (11) und eine koaxial daran anschliessende Gewindepartie (12) umfasst, wobei
C) der Schaft (11) axial verschiebbar in der Hülse (3) gelagert ist,
**dadurch gekennzeichnet, dass**
D) die Gewindepartie (12) ein mehrgängiges Gewinde (13) aufweist; und
E) der Schaft (11) der Ankerschraube (10) und die Hülse (3) der Winkelplatte (1) mit Mitteln (14,4) zur gegenseitigen Rotationssicherung unter Beibehaltung der axialen Verschiebbarkeit versehen sind.

2. Vorrichtung nach Anspruch 1, **dadurch gekennzeichnet, dass** das Gewinde (13) viergängig ist.

3. Vorrichtung nach Anspruch 1 oder 2, **dadurch gekennzeichnet, dass** das Gewinde (13) eine Steigung von mindestens 50 mm, vorzugsweise mindestens 80 mm aufweist.

4. Vorrichtung nach einem der Ansprüche 1 bis 3 **dadurch gekennzeichnet, dass** die Gewindepartie (12) aus einem Kern (16) und spiralförmig um den Kern (16) laufenden Flügeln (17) besteht.

5. Vorrichtung nach einem der Ansprüche 1 bis 4, **dadurch gekennzeichnet, dass** die Querschnittsfläche der Gewindepartie (12) höchstens 55 mm², vorzugsweise höchstens 35 mm² beträgt.

6. Vorrichtung nach einem der Ansprüche 1 bis 4, **dadurch gekennzeichnet, dass** die Querschnittsfläche der Gewindepartie (12) mindestens 10 mm², vorzugsweise mindestens 20 mm² beträgt.

7. Vorrichtung nach einem der Ansprüche 4 bis 6, **dadurch gekennzeichnet, dass** die Flügel (17) eine Dicke von maximal 2,0 mm, vorzugsweise von maximal 1,2 mm aufweisen.

8. Vorrichtung nach einem der Ansprüche 4 bis 6, **dadurch gekennzeichnet, dass** die Flügel (17) eine Dicke von mindestens 0,5 mm, vorzugsweise von mindestens 0,8 mm aufweisen.

9. Vorrichtung nach einem der Ansprüche 1 bis 8, **dadurch gekennzeichnet, dass** der Aussendurchmesser des Gewindes (13) 10 - 14 mm beträgt.

10. Vorrichtung nach Anspruch 9, **dadurch gekennzeichnet, dass** der Aussendurchmesser des Gewindes (13) 11 - 13 mm beträgt.

11. Vorrichtung nach Anspruch 8, **dadurch gekennzeichnet, dass** die Mittel (14,4) aus einer oder mehreren axial auf dem Schaft (11) verlaufenden Längsrillen (14) und damit korrespondierenden, axial in der Hülse (3) verlaufenden Nuten (4) bestehen.

## Claims

1. Device for internal fixation of a femoral head having
A) an angular plate (1) comprising a bone plate (2) configured and dimensioned for attachment to the femur and a sleeve (3) connected at an angle to the bone plate (2);
B) an anchor screw (10) comprising a shaft (11) configured and dimensioned for insertion into the sleeve (3) and a threaded section (12) adjoining coaxially to the shaft (11), whereby
C) the shaft (11) is coaxially displaceable within the sleeve (3),
**characterised in that**
D) the threaded section (12) is provided with a multiple pitch thread (13); and
E) the shaft (11) of the bone screw (10) and the sleeve (3) of the angular plate (1) are provided with means (14;4) apt to prevent mutual rotation while allowing axial movement between the two.

2. Device according to claim 1, **characterised in that** the thread (13) is a four pitch thread.

3. Device according to claim 1 or 2, **characterised in that** the thread (13) is provided with a pitch of at least 50 mm, preferably at least 80 mm.

4. Device according to one of the claims 1 to 3, **characterised in that** the threaded section (12) comprises a core (16) and wings (17) forming a spiral around the core (16).

5. Device according to one of the claims 1 to 4, **characterised in that** the cross-sectional area of the threaded section (12) is not greater than 55 mm², preferably not greater than 35 mm².

6. Device according to one of the claims 1 to 4, **characterised in that** the cross-sectional area of the threaded section (12) is at least 10 mm², preferably at least 20 mm².

7. Device according to one of the claims 4 to 6, **characterised in that** the wings (17) have a maximum thickness of 2,0 mm, preferably of 1,2 mm.

8. Device according to one of the claims 4 to 6, **characterised in that** the wings (17) have a minimum thickness of 0,5 mm, preferably of 0,8 mm.

9. Device according to one of the claims 1 to 8, **characterised in that** the outside diameter of the thread (13) is 10 - 14 mm.

10. Device according to claim 9, **characterised in that** the outside diameter of the thread (13) is 11 - 13 mm.

11. Device according to claim 1, **characterised in that** the means (14;4) comprise one or several longitudinal ridges (14) running axially on the shaft (11) and complementary grooves (4) running axially in the sleeve (3).

## Revendications

1. Dispositif de fixation de têtes d'articulation de hanche fracturées comprenant
A) une plaque coudée (1) qui comporte une plaque (2) pour os pouvant être fixée au fémur et une douille (3) se raccordant de manière angulaire à la plaque pour os,
B) une vis d'ancrage (10) qui comporte une tige (11) pouvant être insérée dans la douille (3) et une partie filetée s'y raccordant coaxialement,
C) la tige étant placée de manière axialement mobile dans la douille (3),
**caractérisé**
D) en ce que la partie filetée (12) présente un filetage (13) à filet multiple, et
E) la tige (11) de la vis d'ancrage (10) et la douille (3) de la plaque coudée (1) sont pourvues de moyens pour la protection réciproque contre la rotation tout en conservant la mobilité axiale.

2. Dispositif selon la revendication 1, **caractérisé en ce que** le filetage (13) est à quadruple filet.

3. Dispositif selon la revendication 1 ou 2, **caractérisé en ce que** le filetage (13) présente un pas d'au moins 50 mm, de préférence d'au moins 30 mm.

4. Dispositif selon une des revendications 1 à 3, **caractérisé en ce que** la partie filetée (12) est constituée d'un noyau (16) et d'ailes (17) s'étendant en forme de spirale autour du noyau (16).

5. Dispositif selon une des revendications 1 à 4, **caractérisé en ce que** la surface de section de la partie filetée (12) est au plus de 55 mm², de préférence au plus de 35 mm².

6. Dispositif selon une des revendications 1 à 4, **caractérisé en ce que** la surface de section de la partie filetée (12) est au moins de 10 mm², de préférence au moins de 20 mm².

7. Dispositif selon une des revendications 1 à 6, **caractérisé en ce que** les ailes (17) présentent une épaisseur au maximum de 2,0 mm, de préférence au maximum de 1,2 mm.

8. Dispositif selon une des revendications 1 à 6, **caractérisé en ce que** les ailes (17) présentent une épaisseur d'au moins 0,5 mm, de préférence d'au moins 0,8 mm.

9. Dispositif selon une des revendications 1 à 8, **caractérisé en ce que** le diamètre extérieur du filetage (13) est de 10 - 14 mm.

10. Dispositif selon la revendication 9, **caractérisé en ce que** le diamètre extérieur du filetage (13) est de 11 - 13 mm.

11. Dispositif selon la revendication 8, **caractérisé en ce que** les moyens (14, 4) sont constitués d'une ou plusieurs stries longitudinales (14) s'étendant axialement sur la tige (11) et de rainures (4) correspondantes s'étendant axialement dans la douille (3).
